# EUROPÄISCHE PATENTSCHRIFT

(11) **EP 2 661 178 B1**
(45) Veröffentlichungstag und Bekanntmachung des Hinweises auf die Patenterteilung: **30.11.2016**
(21) Anmeldenummer: 12712919.5
(22) Anmeldetag: 01.02.2012
(51) Int. Cl.: A61K 9/20, A61K 31/375, A23L 29/00, A23L 33/10, A23P 10/28

(54) **TABLETTIERHILFSMITTEL**
TABLETING AID
AIDE DE COMPRESSION

(30) Priorität: 04.02.2011 DE 102011010437
(43) Veröffentlichungstag der Anmeldung: 13.11.2013
(73) Patentinhaber: J. Rettenmaier & Söhne GmbH + Co. KG, 73494 Rosenberg (DE)
(72) Erfinder: VOLLMER, Reinhard, 55452 Windesheim (DE); GOETZ, Tobias, 73494 Rosenberg (DE)
(74) Vertreter: Dr. Weitzel & Partner
(86) Internationale Anmeldenummer: PCT/EP2012/000441
(87) Internationale Veröffentlichungsnummer: WO 2012/104075

(56) Entgegenhaltungen:
- WO-A1-2010/102841
- DE-A1-102008 014 237
- US-A- 5 725 884
- US-A- 6 106 865
- US-A1- 2003 099 702
- US-A1- 2008 254 119

## Beschreibung

Im Bereich der Pharmazeutischen Technologie ist die Tablette die häufigste Form zur Verpressung von Wirkstoffen. Folglich gibt es in der Pharmazie ein breites Wissen und viel Erfahrung in der Tablettenherstellung. Zusätzlich ist dort eine große Auswahl an Hilfsstoffen zur Herstellung von Tabletten, Granulaten und anderen Festen Formen verfügbar.

Die Hilfsstoffe zur Herstellung von Arzneimitteln sind im Allgemeinen in amtlichen Arzneibüchern (Pharmacopöen) definiert und beschrieben. Es gibt Bindemittel, Formentrennmittel, Schmiermittel, Zerfatlsbeschleuniger, Lösungsvermittler und viele andere.

Neben den Arzneimittel haben sich in den letzten Jahren sogenannte Nahrungsergänzungsmittel etabliert. Die Nahrungsergänzungsmittel sollen Defizite an essentiellen Nährstoffen ausgleichen, die dem menschlichen Körper unter Umständen nicht oder nicht ausreichend zur Verfügung gestellt werden.

Zu den Nahrungsergänzungsmitteln zählen Mineralstoff- und Vitaminpräparate, Zubereitungen von Aminosäuren, Enzyme, Spurenelementen sowie diverse pflanzliche Extrakte.

Nahrungsergänzungsmittel unterliegen in den meisten Ländern der Gesetzgebung für Lebensmittel. Entsprechend ist die Liste der erlaubten Hilfsstoffe zur Verpressung von Tabletten sehr beschränkt. Diese Hilfsstoffe werden zum Beispiel in der RICHTLINIE 96/77/EG DER KOMMISSION genannt. Hingegen sind dort einige Zusatzstoffe nicht aufgeführt, somit für Nahrungsergänzungsmittel und Lebensmittel nicht erlaubt, wohl aber für pharmazeutische Anwendungen.

DE 10 2008 014 237 A1 offenbart ein direkt verpressbares Tablettierhilfsmittel, umfassend eine mikrokristalline Cellulose, Siliziumdioxid, Natriumcarboxymethylstärke sowie Natriumstearylfumarat.

Es wäre außerordentlich wünschenswert, ein direkt verpressbares Tablettierhilfsmittel zu schaffen, das die Vorteile jener Täblettierhilfsmittel aufweiset, die im Bereich der pharmazeutischen Technologie mit Erfolg angewandt werden, und die gleichzeitig für den Einsatz in Lebensmitteln und Nahrungsergänzungsmitteln zulassungsfähig sind. Die gewünschten Eigenschaften sind im Wesentlichen die Folgenden:
- große Tablettenhärte
- gute Schmiereigenschaften
- unkritisch gegenüber langen Mischdauern

Bekannte Tablettierhilfsmittel enthalten in der Regel Einzelstoffe, die jeweils eine der genannten Eigenschaften herbeiführen. Bisher müssen diese Einzelstoffe dem Wirkstoff zudosiert werden, häufig in einer bestimmten Sequenz oder in bestimmten zeitlichen Abständen.

Es gibt zwar Einzelstoffe, die die notwendigen Eigenschaften aufweisen, und die auch den Anforderungen des Lebensmittelrechtes genügen. Sie sind jedoch nicht geeignet, in einfacher Weise mit allen notwendigen anderen Einzelstoffen vereinigt zu werden. So ist beispielsweise Magnesiumstearat ein vorzügliches Schmiermittel, und auch im Lebensmittelbereich zugelassen. Es lässt sich jedoch nicht bei Raumtemperatur ausreichend emulgieren, sondern müsste erst über den Schmelzpunkt aufgeteilt und in flüssiger Form zugegeben werden, was energetisch aufwendig ist und sonstige Probleme nach sich zieht.

Der Erfindung liegt somit die Aufgabe zugrunde, ein direkt verpressbares Tablettierhilfsmittel sowie ein Verfahren zu dessen Herstellung anzugeben, womit die gleichen oder ähnlichen Eigenschaften wie bei einem pharmazeutischen Tablettierhilfsmittel erzielt werden können, aber gleichzeitig den Anforderungen des Lebensmittelrechtes genügt wird. Ganz wichtig ist aber die Teilaufgabe, Tablettierhilfsstoffe zu finden, die sich einem Co-Processing unterwerfen lassen, bei dem die Ausgangsstoffe bei Raumtemperatur oder nur wenig darüber vorgelegt werden, zumindest ohne die Notwendigkeit des Erhitzens auf dreistellige Temperaturgrade, um ein fertiges Compound zu erzielen, das nur noch dem Wirkstoff der herzustellenden Tablette zugegeben werden muss.

Diese Aufgabe wird durch die Merkmale der selbstständigen Ansprüche gelöst,

Das erfindungsgemäße Tablettierhiffsmittel enthält somit als notwendige Bestandteile ein Bindemittel wie mikrokristalline Cellulose, einen Emulgator, wie beispielsweise Lecithin sowie ein pflanzliches oder tierisches Fett als Schmiermittel. Ein solches Tablettierhilfsmittel weist die folgenden Vorzüge auf: Es entspricht den Anforderungen des Lebensmittelrechtes; es enthält alle notwendigen Einzelstoffe, die die gewünschten, zum Tablettieren erforderlichen Eigenschaften aufweist; das Co-Processing lässt sich durchrühren bei Raumtemperatur oder nur wenig erhöhter Temperatur, somit praktisch ohne Energieaufwand, so dass ein kompletter Tablettierhilfsstoff entsteht, der mit dem Wirkstoff der Tablette nur noch gemischt werden muss.

Aus WO 2009/112287 A1 ist die Verwendung von Natriumstearylfumarat als Schmiermittel bekannt. Aufgrund seiner chemischen Struktur wirkt Natriumstearylfumarat nicht nur als Schmiermitteln, sondern auch als Emulgator.

Im Lebensmittelbereich ist dies aber nicht zugelassen. Mit der erfindungsgemäßen Kombination Lecithin/Pflanzenfett wurde jedoch ein Compound gefunden, das als Schmiermittel und als Emulgator wirkt, und das außerdem lebensmittelrechtlich zugelassen ist.

Die oben genannten Hilfsstoffe werden in der unten beschriebenen Weise derart miteinander verbunden, dass ein Compound zur direkten Verpressung von Tabletten und Granulaten entsteht.

Das erfindungsgemäße Compound weist die folgernden Vorteile gegenüber der physikalischen Mischung von Einzelkomponenten auf:
1. Durch die Fixierung der schmierenden Substanzen - gehärtetes Pflanzenfett und Lecithin - an das Bindemittel Mikrokristalline Cellulose, werden während des Mischprozesses die bindenden Teilchen nicht mehr gefettet. Das ergibt bei der Verpressung eine deutlich höhere Tablettenhärte als mit der physikalischen Mischung.
2. Die Konzentration an den fixierten, schmierenden Stoffen reicht aus, um während des Pressvorgangs die Matrize der Tablettenpresse ausreichend zu schmieren. Eine zusätzliche Zugabe von Schmiermitteln wie zum Beispiel Magnesiumstearat entfällt.
3. Dank der gleichmäßigen Verteilung des fixierten Schmiermittels sind die Ausstoßkräfte für die Tablette geringer.
4. Längere Mischzeiten haben keinen negativen Einfluss auf die Tablettenhärte. Es können auch schlecht mischbare Stoffe ausreichend lange gemischt werden.
5. Der wesentliche Vorteil der Erfindung liegt darin, dass der Wirkstoff nur noch mit dem Tablettierhilfsmittel gemischt werden muss. Die Verpressung kann sofort anschließend erfolgen. Es werden keine weiteren Zusatzstoffe benötigt.

Die Herstellung des beschriebenen Compounds aus den oben genannten Komponenten erfolgt vorzugsweise durch Nassgranulierung nach allen bekannten Methoden wie der Mischgranulierung, Lochscheibengranulierung, Wirbelschichtgranulierung, Extrusion oder Shugi-Granulation.

Eine Granulierung in der Wirbelschicht kann beispielsweise wir folgt ablaufen:
I. Im Wirbelschichttrockner werden silicifizierte mikrokristalline Cellulose und quervernetzte Natrium Carboxymethylcellulose vorgelegt. Die Mischung wird auf 30 - 35 °C erwärmt. Parallel werden 10 Teile Kokosfett, 10 Teile Lecithin und 100 Teile Wasser mit hohen Scherkräften emulgiert. Die entstandene Emulsion wird langsam über eine Top-spray Düse auf die vorgelegte Cellulose aufgesprüht. Das Material wird im Luftstrom auf eine Feuchte von 3,0 - 3,5 % getrocknet.
II. Mikrokristalline Cellulose und vernetzte Natrium Carboxymethylcellulose werden im Wirbelschichttrockner vorgelegt. Parallel werden 10 Teile Kokosfett, 10 Teile Lecithin, 2 Teile Kolloidales Siliciumdioxid und 98 Teile Wasser mit hohen Scherkräften emulgiert. Die entstandene Emulsion wird langsam über eine Top-spray Düse auf die vorgelegte Cellulose ausgesprüht Das Material wird im Luftstrom auf eine Feuchte von 3,0 - 3,5% getrocknet. In beiden Fällen resultiert ein freifließendes direkt verpressbares Pulver.

### Tablettierversuche

Das hergestellte Compound lässt sich nicht direkt mit der physikalischen Mischung der Einzelbestandteile vergleichen, da sich das Pflanzenfett nicht pur mit der mikrokristallinen Cellulose vermischen lässt. Daher wurden klassische Formulierungen unter Verwendung von reiner mikrokristallinen Cellulose als Bindemittel und Magnesiumstearat als Schmiermittel verglichen.

Beispiel Acsorbinsäure-Tablette:

| Zutaten | Verhältnis Wirkstoff zu Hilfsstoff | | | klassische Mischung |
|---|---|---|---|---|
| | | | | |
| Ascorbinsäure | 50 % | | | 50 % |
| Mikrokristalline Cellulose | | | | 45 % |
| Siliciumdioxid | | | | 2 % |
| Na-Carboxymethylcellulose, quervernetzt | | | | 2 % |
| Magnesiumstearat | | | | 1 % |
| Tablettferhilfsmittel | 50 % | | | |
| | | | | |

| Prüfparameter | | Tablettierhilfsmittel | klassische Mischung | |
|---|---|---|---|---|
| | | | | |
| Presskraft ( kN ) | | 7,0 - 10,0 | 7,0 - 10,0 | |
| Ausstoßkraft ( N ) | | 90 - 95 | 110 - 130 | |
| Tablettenhärte ( N ) | | 120 - 170 | 90 - 110 | |
| Zerfallszeit ( sec ) | | 30 - 40 | 30 - 40 | |

Beispiel Pflanzenextrakt Tablette:

| Zutaten | Tablettierhilfsmittel | klassische Mischung |
|---|---|---|
| | | |
| Pflanzenextrakt | 50 % | 50 % |
| Mikrokristalline Cellulose | | 45 % |
| Siliciumdioxid | | 2 % |
| Na-Carboxymethylcellulose, quervernetzt | | 2 % |
| Magnesiumstearat | | 1 % |
| Tablettierhilfsmittel | 50 % | |
| | | |

| Prüfparameter | Tablettierhilfsmittel | klassische Mischung |
|---|---|---|
| | | |
| Preßkraft ( kN ) | 7,0 - 10.00 | 7,0 - 10,0 |
| Ausstoßkraft ( N ) | 50 - 70 | 100 - 120 |
| Tablettenhärte ( N ) | 110 - 140 | 100 - 115 |
| Zerfallszeit ( sec ) | 60 - 80 | 70 - 100 |

Das erfindungsgemäße Tablettierhilfsmittel lässt sich mit vielen im Bereich der Nahrungsergänzungsmittel üblicherweise verwendeten Wirkstoffen verpressen. Das sind Vitamine, rein oder in Mischungen, Mineralstoffe, rein oder in Mischungen, Pflanzenextrakte, Enzyme, Aminosäuren sowie andere Substanzen wie zum Beispiel Glucosamin, Condroitin, Methylsulfonyl Sulfat und weitere.

## Patentansprüche

1. Direktverpressbares Tablettierhilfsmittel für den Einsatz in Lebensmitteln (Nahrungs-ergänzungsmitteln) enthaltend:
78 - 97 % einer mikrokristallinen Cellulose als Emulgator Lecithin in der Dosierung 0,1 - 5,0 %.
0,1-5,0 % gesättigtes Pflanzenfett
0,5 -10 % kolloidales Siliziumdioxid als Fließgulierungsmittel
0,5-5% Natrium Carboxymethylcellulose als Zerfallsbeschleuniger

2. Verfahren zum Herstellen eines direkt verpressbaren Tablettierhilfsmittels, wenigstens umfassend die Stoffe gemäß Anspruch 1, **gekennzeichnet durch** die folgenden Verfahrensschritte:
es wird eine Naßgranulierung vorgenommen, umfassend eine Mischgranulierung oder eine Lochscheibengranulierung oder eine
Wirbelschichtgranulierung oder eine Extrusion oder eine Shugi-Granulierung oder eine Sprühtrocknung.

3. Verfahren nach Anspruch 2, **gekennzeichnet durch** eine Wirbelschichtgranulierung mit den folgenden Verfahrensschritten:
- Vorlegen einer silizifizierten mikrokristallinen Cellulose und einer quervernetzten Natrium Carboxymethylcellulose
- Erwärmen des Gemisches auf eine Temperatur von 20 bis 40° C
- Emulgieren von 10 Teilen von Kokosfett, Lecithin und Wasser unter Anwendung hoher Scherkräfte
- Aufsprühen der entstandenen Emulsion auf die vorgelegte Cellulose
- Trocknen im Luftstrom auf eine Feuchte von 2 - 5 %.

## Claims

1. A directly compressible tableting excipient for use in foods (dietary supplements), containing:
78 to 97% of a microcrystalline cellulose,
a lecithin in the dosage of 0.1 to 5.0% as an emulsifier,
0.1 to 5.0% of a saturated vegetable fat,
0.5 to 10% of a colloidal silicon dioxide as a glidant,
0.5 to 5% of sodium carboxymethyl cellulose is a disintegrant.

2. A method for producing a directly compressible tableting excipient, at least comprising the substances according to claim 1, **characterized by** the following method steps:
performing a wet granulation, comprising mixing granulation or perforated-disc granulation or
fluidized-bed granulation or extrusion or Shugi granulation or spray drying.

3. A method according to claim 2, **characterized by** fluidized-bed granulation with the following method steps:
- providing a microcrystalline cellulose and a cross-linked sodium carboxymethyl cellulose,
- heating the mixture to a temperature of 20 to 40°C,
- emulsifying 10 parts of coconut oil, lecithin and water under application of high shearing forces,
- spraying the obtained emulsion onto the provided cellulose,
- drying in the air flow to a humidity of 2 to 5%.

## Revendications

1. Adjuvant pour comprimés compressible directement pour la préparation de produits alimentaires (compléments alimentaires), contenant :
de 78 à 97 % d'une cellulose microcristalline,
de la lécithine servant d'émulsifiant dosée entre 0,1 % et 5,0 %,
de 0,1 % à 5,0 % de matière grasse saturée d'origine végétale,
de 0,5 % à 10 % de dioxyde de silicium colloïdal servant de fluidifiant,
de 0,5 % à 5 % de carboxyméthylcellulose sodique servant d'accélérateur de dissolution.

2. Procédé pour la fabrication d'un adjuvant pour comprimés compressible directement et contenant au moins les substances énumérées dans la revendication 1, **caractérisé en ce qu'**il comprend les étapes suivantes :
réalisation d'une granulation par voie humide, comprenant une granulation en mélange ou une granulation sur plaque perforée ou une granulation en lit fluidisé ou une extrusion ou une granulation dans un mélangeur Shugi ou un séchage par pulvérisation.

3. Procédé selon la revendication 2, **caractérisé en ce qu'**il comprend une granulation en lit fluidisé avec les étapes suivantes :
- préparation d'une cellulose microcristalline silicifiée et d'une carboxyméthylcellulose sodique réticulée ;
- chauffage du mélange à une température de 20 à 40 °C ;
- émulsification de 10 parts de graisse de coco, de lécithine et d'eau en appliquant des forces de cisaillement élevées ;
- pulvérisation de l'émulsion obtenue sur la cellulose préparée ;
- séchage dans un flux d'air jusqu'à une humidité de 2 % à 5 %.
